# EUROPÄISCHE PATENTANMELDUNG

(11) **EP 0 580 971 A1**
(43) Veröffentlichungstag der Anmeldung: **02.02.1994**
(21) Anmeldenummer: 93107574.1
(22) Anmeldetag: 10.05.1993
(51) Int. Cl.: A61B 19/02, B65F 1/06

(54) **Sterilisierbarer Behälter**

(30) Priorität: 31.07.1992 DE 9210307 U
(71) Anmelder: MMM MÜNCHENER MEDIZIN MECHANIK GMBH, D-81369 München (DE)
(72) Erfinder: Goder, Franz, W-8000 München 70 (DE)
(74) Vertreter: Grünecker, Kinkeldey, Stockmair & Schwanhäusser Anwaltssozietät

(57) **Zusammenfassung**

Die Erfindung bezieht sich auf einen in eine Desinfektions- bzw. Sterilisierkammer einbringbaren Behälter zur Desinfektion bzw. Sterilisation von darin enthaltenem Desinfizier- bzw. Sterilisiergut, mit einem Aufnahmeteil (1) mit obenliegender Öffnung und einem Deckel (2) zum Verschließen der Öffnung, in welchem ein in zwei entgegengesetzte Richtungen öffnendes Ventil (7) angeordnet ist, das bei Auftreten einer Druckdifferenz zwischen Äußerem und Innerem des geschlossenen Behälters öffnet und in Offenstellung das Behälterinnere mit der Umgebung des Behälters verbindet. Der Erfindung liegt die Aufgabe zugrunde, einen derartigen Behälter zu schaffen, in welchen auch große Mengen und/oder große Objekte von Desinfizier- bzw. Sterilisiergut eingebracht werden können, der einfach und ohne Kontaminationsgefahr für das Bedienungspersonal handhabbar ist, der in gefülltem und geschlossenem Zustand einem Desinfizier- bzw. einem Sterilisationsverfahren zuführbar ist und eine einfache Entnahme und wirtschaftliche Entsorgung ermöglicht. Bei dem erfindungsgemäßen Behälter wird diese Aufgabe dadurch gelöst, daß der Aufnahmeteil (1) eine zur Aufnahme eines Müllsackes (3) marktüblichen Fassungsvermögens geeignete tonnenförmige Gestalt und Dimensionierung aufweist, und daß innerhalb des Aufnahmeteils (1), in dessen oberem Bereich unterhalb des Randes der Öffnung eine Einhängevorrichtung (4) vorgesehen ist, in welche der Müllsack (3) ohne Überstehen von Randteilen der Müllsacköffnung über den Rand der Öffnung des Aufnahmeteils (1) einhängbar ist.

## Beschreibung

Die Erfindung bezieht sich auf einen Behälter gemäß dem Oberbegriff von Anspruch 1.

Aus dem deutschen Gebrauchsmuster G 87 17 433.2 ist ein Behälter der eingangs genannten Art bekannt, bei dem es sich um eine nahezu quaderförmige Schachtel mit Klappdeckel handelt, in welche ein Beutel mittels eines Spannringes eingehängt werden kann. Diese Schachtel wird insbesondere zur Sterilisation von chirurgischem Besteck verwendet und ist in ihrer Kapazität begrenzt und zur Aufnahme großer Mengen und ggf. großer Objekte an Sterilisiergut nicht geeignet.

In der deutschen Patentschrift DE C2 26 10 290 ist ein Verschlußventil für einen filterlosen Sterilisierbehälter beschrieben, das sich bei Funktionsstörungen besonders einfach zerlegen läßt. Angaben zu einem Einbau des Ventiles in einen Behälter sind in der Patentschrift nicht enthalten.

Der Erfindung liegt die Aufgabe zugrunde, einen Behälter der eingangs angegebenen Art zu schaffen, in welchen auch große Mengen und/oder große Objekte von Desinfizier- bzw. Sterilisiergut eingebracht werden können, der einfach und ohne Kontaminationsgefahr für das Bedienungspersonal handhabbar ist, der in gefülltem und geschlossenem Zustand einem Desinfektionsbzw. Sterilisationsverfahren zuführbar ist und eine einfache Entnahme und wirtschaftliche Entsorgung ermöglicht.

Erfindungsgemäß wird diese Aufgabe dadurch gelöst, daß der Aufnahmeteil eine zur Aufnahme eines Müllsackes marktüblichen Fassungsvermögens geeignete tonnenförmige Gestalt und Dimensionierung aufweist, und daß innerhalb des Aufnahmeteils, in dessen oberem Bereich unterhalb des Randes der Öffnung eine Einhängevorrichtung vorgesehen ist, in welche der Müllsack, ohne Überstehen von Randteilen der Müllsacköffnung über den Rand der Öffnung des Aufnahmeteiles einhängbar ist.

Dadurch wird es auf vorteilhafte Weise möglich, auch große Mengen und ggf. große Objekte von Desinfizier- bzw Sterilisiergut in einem verschließbaren und einfach handhabbaren Behälter kontaminationssicher zu erfassen und darin zu desinfizieren bzw. zu sterilisieren und den desinfizierten bzw. sterilisierten Müll ggf. bis zum Abtransport im Behälter zu belassen, um den Müllsack vor Beschädigung von außen zu schützen bzw. um die Gefahr einer Verletzung an scharfkantigen, die Sackwand durchstoßenden Gegenständen zu vermeiden. Der erfindungsgemäße Behälter ist wegen der Ventilanordnung im Deckel auch in Verfahren der fraktionierten Desinfektion/Sterilisation ohne weiteres einsetzbar.

Eine günstige konstruktive Ausbildung des Behälters ist dadurch gegeben, daß eine Einhängevorrichtung einen in dem Aufnahmeteil gehalterten Ring aufweist, in den ein Müllsack unter Umstülpen seines oberen Randbereiches entlang des gesamten Ringumfanges einhängbar ist. Auf diese Weise wird vermieden, daß der Rand der Öffnung des Müllsackes in eine Dichtfläche zwischen Aufnahmeteil und Deckel hinein- bzw. hindurchragt. Zusätzlich ist dieser Sackrand vor Verunreinigungen geschützt und ermöglicht dadurch insbesondere auch beim Verschließen des Sackes eine sichere Handhabung.

Eine besonders einfache Handhabung des Behälters insbesondere die Entnahme des Müllsackes wird dadurch erreicht, daß der Ring der Einhängevorrichtung schwenkbar gelagert ist, und zur Entnahme des Müllsackes aufgeschwenkt werden kann. Durch die schwenkbare Lagerung ist der Ring zugleich gegen Verlust gesichert.

Eine zum Schutz eines Druck-Vakuumventiles besonders günstige Ausgestaltung des Behälters ist dadurch gegeben, daß eine lochplatte im Bereich des Ventiles im Deckel des Behälters vorgesehen ist, welche insbesondere bei Überfüllung des Aufnahmeteils oder Zerplatzen von Hohlkörpern bei der Durchführung des Desinfektions- bzw. Sterilisationsverfahrens, das Druck-Vakuumventil vor Beschädigung schützt.

Eine vorteilhafte Ausbildung des Behälters ist dadurch gegeben, daß ein Verschlußmechanismus den Aufnahmeteil und den Deckel in Schließstellung verbindet und dadurch in besonders einfacher Weise eine ggf. für eine Dichtvorrichtung erforderliche Schließkraft erzeugt und es ermöglicht, den Behälter vor unbefugtem Öffnen zu sichern.

Eine zur Vermeidung von Gasdiffusion zwischen Deckel und Aufnahmeteil in Schließstellung besonders günstige Ausgestaltung des Behälters ist dadurch gegeben, daß im Bereich der Öffnung des Aufnahmeteils als auch an dem dem Rand der Öffnung zugewandten Bereich des Deckels in Schließstellung von Deckel und Aufnahmeteil Zusammenwirkende Dichtelemente vorgesehen sind, welche aus nicht saugfähigen bzw. nicht porösen Weichstoffen gefertigt sind, um festhaftende Verunreinigungen an der Dichtfläche zu vermeiden. Eine besonders vorteilhafte Wirkung ergibt sich aus der sicheren Abdichtung in Verbindung mit der hohen Aufnahmekapazität dahingehend, daß sich die Befüllung des Behälters über einen längeren Zeitraum erstrecken kann, und dadurch der Behälter insbesondere als Sammelbehälter zu verwenden ist.

Eine besonders günstige Ausbildung des Behälters ist auch dadurch gegeben, daß ein Rand des Deckels über den Umfang des Aufnahmeteils des Behälters und über eine daran zum Verbinden von Aufnahmeteil und Deckel befestigte Verschlußvorrichtung hinausragt, dadurch ist der Verschlußmechanismus vor Beschädigung geschützt.

Eine für den Transport des Behälters besonders günstige Ausgestaltung wird dadurch erreicht, daß der Behälter fahrbar ausgebildet ist, eine Ausführung mit Zwei Rädern und mindestens einer Stütze ermöglicht ein standfestes Abstellen des Behälters ohne zusätzliches Feststellen der Räder.

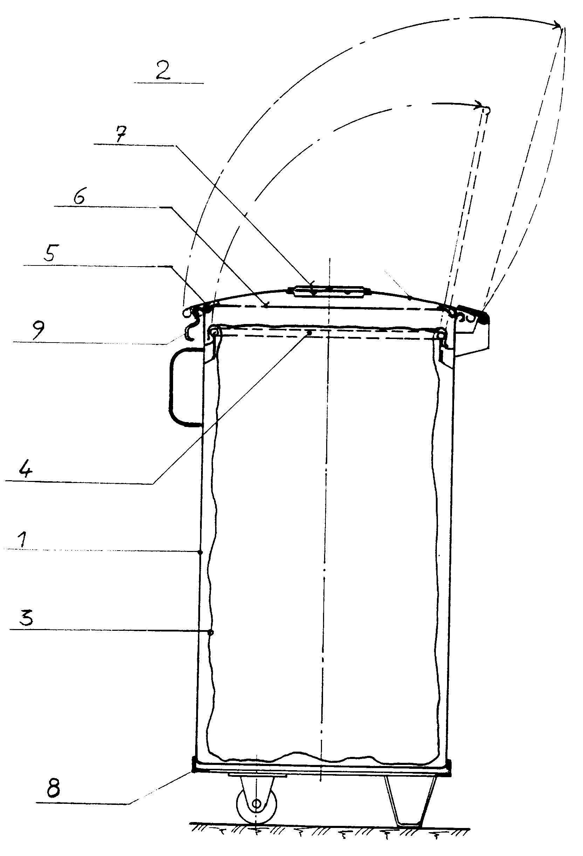

Weitere Einzelheiten und Vorteile der Erfindung ergeben sich aus der nachfolgenden Beschreibung eines Ausführungsbeispieles in Verbindung mit der Zeichnung.

Die einzige Figur der Zeichnung zeigt
einen Längsschnitt durch ein Ausführungsbeispiel des erfindungsgemäßen Behälters.

Der Behälter verfügt über einen tonnenförmigen Aufnahmeteil 1 mit einem innenliegenden Müllsack 3 marktüblichen Fassungsvermögens zur Aufnahme von Desinfektions- bzw. Sterilisiergut. Der Müllsack 3 ist an einer innenliegenden Einhängevorrichtung 4 unter Umstülpen seines oberen Randbereiches entlang des gesamten Umfanges eines Ringes einhängbar. Durch das Einhängen des Sackrandes innerhalb des Behälters 1 wird dieser Sackrand vor Verunreinigungen geschützt. Durch diesen unverschmutzten Sackrand wird eine sichere Handhabung des Müllsackes 3 insbesondere bei der Entnahme ermöglicht.

Der Aufnahmeteil 1 wird durch einen schwenkbar gehalterten Deckel 2 verschlossen. Ein Druck-Vakuumventil 7 ist vorzugsweise im Deckel 2 des Behälters eingebaut. Das Druck-Vakuumventil 7 öffnet während des Desinfektions- bzw. Sterilisationsverfahrens, bzw. bei Druckdifferenzen außerhalb des durch die Bauart des DruckVakuumventiles 7 festgelegten Schließdruckbereiches, und verbindet bei geschlossenem Behälter in Offenstellung das Behälterinnere mit der Umgebung des Behälters. Das Druck-Vakuumventil 7 wird durch eine Lochplatte 6 vor mechanischer Beschädigung geschützt.

Eine Dichtvorrichtung 5 an dem dem Rand der Öffnung zugewandten Bereich des Deckels 2, dichtet in Schließstellung unter ZusammenWirkung mit dem Randbereich des Aufnahmeteils 1 den Behälter ab. Eine besonders sichere Dichtwirkung durch die Dichtvorrichtung 5 wird insbesondere dadurch erreicht, daß kein Sackrand in die Dichtfläche zwischen Aufnahmeteil 1 und Deckel 2 hineinragt. In Strichlinien ist die Position von Deckel 2 bzw. Einhängevorrichtung 4 in geschwenktem Zustand dargestellt. Aufnahmeteil 1 und Deckel 2 werden mittels mindestens eines Verschlußmechanismus 9 in Schließstellung gehalten. Durch Überkragen des Randes des Deckels 2 wird in vorteilhafter Weise der Verschlußmechanismus 9 überragt.

Der Aufnahmeteil 1 ist mit einem fahrbaren Untersatz 8 verbunden um insbesondere die Handhabung des Behälters in gefülltem Zustand zu verbessern.

Der vorstehend beschriebene Behälter kann beispielsweise in folgender Weise eingesetzt werden: In einem Krankenhaus wird von einem Operationssaal kommender kontaminierter Abfall nach Öffnen des Deckels des Behälters in den Behälter eingebracht. Nach Einbringung des kontaminierten Abfalles wird der Behälter verschlossen. Durch sukzessives Einbringen von kontaminierten Abfällen wird der Behälter gefüllt. Der gefüllte Behälter wird verschlossen in eine VakuumSterilisierkammer eingebracht und dem Sterilisierverfahren unterzogen. Nach Beendigung des Sterilisierverfahrens wird der Behälter mit dem sterilisierten Inhalt der Sterilisierkammer entnommen. Der Behälter bleibt bis zur Entnahme des Müllsackes verschlossen. Zum Abtransport des Müllsackes wird der Behälter geöffnet, der Ring der Einhängevorrichtung geschwenkt, der Müllsack verschlossen und aus dem Aufnahmeteil herausgenommen. Der sterilisierte Müll wird im geschlossenen Müllsack einer Deponie oder einer Verbrennungsanlage zugeführt.

Die Erfindung ist nicht auf das vorstehend beschriebene Ausführungsbeispiel beschränkt. Beispielsweise kann der Aufnahmeteil 1 länglich polygonal oder konisch ausgeführt werden.

Sämtliche aus der Beschreibung und der Zeichnung, einschließlich der konstruktiven Einzelheiten, hervorgehenden Merkmale können auch in beliebigen Kombinationen erfindungswesentlich sein.

## Patentansprüche

1. In Desinfektions- bzw. Sterilisierkammer eines Desinfektions- bzw. Sterilisiergerätes einbringbarer Behälter, zur Desinfektion bzw. Sterilisation von darin enthaltenem Desinfizier- bzw. Sterilisiergut, mit einem Aufnahmeteil mit obenliegender Öffnung und einem Deckel zum Verschließen der Öffnung, in welchem ein in zwei entgegengesetzte Richtungen öffnendes Ventil angeordnet ist, das bei Auftreten einer Druckdifferenz zwischen Äußerem und Innerem des geschlossenen Behälters öffnet und in Offenstellung das Behälterinnere mit der Umgebung des Behälters verbindet, **dadurch gekennzeichnet**, daß der Aufnahmeteil (1) eine zur Aufnahme eines Müllsackes (3) marktüblichen Fassungsvermögens geeignete, tonnenförmige Gestalt und Dimensionierung aufweist, und daß innerhalb des Aufnahmeteils (1), in dessen oberem Bereich unterhalb des Randes der Öffnung eine Einhängevorrichtung (4) vorgesehen ist, in welche der Müllsack ohne Überstehen von Randteilen der Müllsacköffnung über den Rand der Öffnung des Aufnahmeteiles (1) einhängbar ist.

2. Behälter nach Anspruch 1, **dadurch gekennzeichnet**, daß die Einhängevorrichtung (4) einen in dem Aufnahmeteil (1) gehalterten Ring aufweist, in den der Müllsack (3) unter Umstülpen seines oberen Randbereiches entlang des gesamten Ringumfanges einhängbar ist.

3. Behälter nach Anspruch 2, **dadurch gekennzeichnet**, daß der Ring im Inneren des Aufnahmeteils (1) schwenkbar gelagert ist.

4. Behälter nach mindestens einem der Ansprüche 1 bis 3, **dadurch gekennzeichnet**, daß an der Unterseite des Deckels (2) zumindest im Bereich des Ventiles (7) eine Lochplatte (6) als Spritzschutz angebracht ist.

5. Behälter nach mindestens einem der Ansprüche 1 bis 4, **dadurch gekennzeichnet**, daß der Aufnahmeteil (1) und der Deckel (2) mittels mindestens eines Verschlußmechanismus (9) miteinander verbindbar sind.

6. Behälter nach mindestens einem der Ansprüche 1 bis 5, **dadurch gekennzeichnet**, daß sowohl im Bereich des Randes der Öffnung des Aufnahmeteils (1) als auch an dem dem Rand der Öffnung zugewandten Bereich des Deckels (2) in Schließstellung von Deckel und Aufnahmeteil zusammenwirkende Dichtelemente (5) vorgesehen sind.

7. Behälter nach mindestens einem der Ansprüche 1 bis 6, **dadurch gekennzeichnet**, daß ein Rand des Deckels (2) über den Umfang des Aufnahmeteils (1) des Behälters und über eine daran zum Verbinden von Aufnahmeteil (1) und Deckel (2) befestigte Verschlußvorrichtung (9) hinausragt.

8. Behälter nach mindestens einem der Ansprüche 1 bis 7, **dadurch gekennzeichnet**, daß der Behälter fahrbar ausgebildet ist.
